# EUROPÄISCHE PATENTSCHRIFT

(11) **EP 1 465 658 B1**
(45) Veröffentlichungstag und Bekanntmachung des Hinweises auf die Patenterteilung: **02.05.2007**
(21) Anmeldenummer: 03708059.5
(22) Anmeldetag: 15.01.2003
(51) Int. Cl.: A61K 39/00, A61K 39/385, A61P 37/00

(54) **ORALE VAKZINIERUNG MIT DEM TUMOR-ANTIGEN-MIMOTOP Gln-Met-Trp-Ala-Pro-Gln-Trp-Gly-Pro-Asp**
ORAL VACCINATION WITH THE TUMOR ANTIGEN MIMOTOPE Gln-Met-Trp-Ala-Pro-Gln-Trp-Gly-Pro-Asp
VACCINATION ORALE AVEC LE MIMOTOPE D'ANTIGENE Gln-Met-Trp-Ala-Pro-Gln-Trp-Gly-Pro-Asp

(30) Priorität: 15.01.2002 EP 02000901; 17.12.2002 DE 10259053
(43) Veröffentlichungstag der Anmeldung: 13.10.2004
(73) Patentinhaber: Bio Life Science Forschungs- und Entwicklungsges.m.b.H., 1010 Wien (AT)
(72) Erfinder: JENSEN-JAROLIM, Erika, A-1210 Wien (AT); SCHEINER, Otto, A-2380 Perchtoldsdorf (AT); PEHAMBERGER, Hubert, A-1230 Wien (AT); ZIELINSKI, Christoph, A-1180 Wien (AT); BREITENEDER, Heimo, A-1070 Wien (AT)
(74) Vertreter: Kador & Partner
(86) Internationale Anmeldenummer: PCT/EP2003/000369
(87) Internationale Veröffentlichungsnummer: WO 2003/059380

(56) Entgegenhaltungen:
- EP-A- 1 327 450
- WO-A-96/33732
- WO-A-98/43479
- WO-A1-01/08495
- WO-A1-99/07869
- US-A- 471 353
- US-A- 5 840 332
- UNTERMAYR ET AL.: "Anaphylaxis to russian beluga caviar" EUROPEAN JOURNAL OF ALLERGY AND IMMUNOLOGY, Bd. 109, Nr. 6, 2002, Seiten 1034-1035, XP008018087 in der Anmeldung erwähnt
- DATABASE [Online] 24 Oktober 2005 'Gastric Acid inhibitors' Retrieved from WWW.SURGERYENCYCLOPEDIA.COM/FI-LA/G ASTRIC-ACID-INHIBITORS.HTML
- LAHEIJ R.J. ET AL: 'Medical biology pneumonia and gastric-acid suppressive drugs' J AM MED ASSOC, [Online] Bd. 292, 2004, Seite 1955 Gefunden im Internet: <URL:http://scienceweek.com/2004/sc041217-2 .htm>>
- Hochschule für angewandte Wissenschaften Hamburg, Fakultät Life Science, Studiengang Ökotrophologie, Anatomie/Physiologie, Klausur im SS2005 am 29.6.2005, Prof. Dr. Lorenz
- GANGLBERGER E. ET AL: 'Monovalent fusion proteins of IgE mimotopes are safe for therapy of type I allergy' THE FASEB JOURNAL Bd. 15, November 2001, Seiten 2524 - 2526
- ZUERCHER A.W. ET AL: 'Oral anti-IgE immunization with epitope-displaying phage' EUROPEAN JOURNAL OF IMMUNOLOGY Bd. 30, Nr. 30, Seiten 128 - 135

## Beschreibung

Die vorliegende Erfindung betrifft ein Arzneimittel zur oralen Vakzinierung gemäß Anspruch 1.

In der Medizin ist es häufig erwünscht, dass Krankheiten nicht nur symptomatisch bekämpft werden, sondern ihr Entstehen von vorne herein verhindert wird. Im Bereich der Infektionskrankheiten, neuerdings aber auch im Bereich der Krebserkrankungen wird daher der Weg der Vakzinierung eingeschlagen. Mittels dieser Methode wird der Körper dazu befähigt, mit Hilfe seines Immunsystems auf krankheitserregende Substanzen, auch Antigene genannt, zu reagieren. Dieses auch als aktive Immunisierung bekannte Verfahren funktioniert dadurch, dass dem Immunsystem ein mit einer Krankheit assoziiertes Antigen präsentiert wird, worauf das Immunsystem mit der Bildung entsprechender spezifischer Antikörper gegen besagte Antigene reagiert. Diese sogenannten Antigene bestehen häufig aus Peptiden oder Proteinen, dass heißt Aminosäuresequenzen, welche häufig parenteral, das heißt unter Umgehung des Magen-Darm-Traktes verabreicht werden. Dies geschieht meist mittels Injektion oder Infusion, was von den Patienten häufig als unangenehm empfunden wird.

US-A-4,171,353 beschreibt die orale Immunisierung von Tieren mittels u.a. Tumorzellen und einem Cholinester als Adjuvans sowie einer Magensäure reduzierenden Substanz, Eur. J. Immunol. 2000, 30, 128-135 beschreibt eine orale anti-IgE Immunisierung mittels Phagendisplay zur Behandlung von Allergien.

Dem gemäß ist es Aufgabe der vorliegenden Erfindung, eine Anti-Tumor-Vakzine zur Verfügung zu stellen, mit Hilfe derer es möglich ist, eine Impfung auf parenteralem Wege zu vermeiden und die Akzeptanz bei den Patienten dadurch zu steigern.

Der Erfindung liegt die Erkenntnis zugrunde, dass eine parenterale Applikation dann umgangen werden kann, wenn im Magenbereich geeignete Bedingungen herrschen, die die Wirksamkeit des Impfstoffes bei Aufnahme über den Magen-Darm-Trakt nicht beeinträchtigen und somit die Bildung von Antikörpern ermöglichen.

Gegenstand der vorliegenden Erfindung sind Arzneimittel gemäß Anspruch 1.

Durch diese Arzneimittel wird zum einen ermöglicht, dass die als Antigen wirksame Substanz gemäß Anspruch 1 gleichzeitig mit der magensäure-reduzierenden Substanz eingenommen werden kann, wobei beide Phasen zusammen oder getrennt vorliegen können. Zum anderen kann das Kombinationspräparat auch in der Weise appliziert werden, dass die als Antigen wirksame Substanz gemäß Anspruch 1 getrennt und nach der Applikation einer geeigneten Dosis an magensäurereduzierender Substanz verabreicht wird. Dabei ist es wichtig, dass die Magensäure zum Zeitpunkt der Applikation, das heißt zu dem Zeitpunkt, an dem der Impfstoff in den Magen gelangt, im Vergleich zum "normalen Magensäuregehalt" reduziert ist. Auf diese Weise wird es ermöglicht, dass eine traditionell parenteral zu verabreichende Vakzine auch oral appliziert werden kann und ihre Wirkung in Form der Erzeugung einer Immunantwort entfalten kann. Insbesondere werden als Immunantwort Immunglobuline der Gruppen IgE und IgG1 erzeugt. Des weiteren hat die reduzierte Azidität auch eine Nicht-Aktivierung der Magen-Proteasen zur Folge, denn Pepsinogene werden erst durch den niedrigen pH im Magen zu den aktiven Pepsinen gespalten.

Da beispielsweise Tiere sowohl Tumoren entwickeln können, als auch in der Lage sind, IgE zu produzieren, ist beispielsweise im veterinärmedizinischen Bereich an eine Vakzinierung gemäß der vorliegenden Erfindung zu denken. Bevorzugterweise dienen die erfindungsgemäßen Arzneimittel daher zur Vakzinierung von Tieren, insbesondere Säugetieren. Besonders bevorzugt dienen die erfindungsgemäßen Arnzeimittel zur Vakzinierung von Menschen.

Beispiele für solche Substanzen sind Krebsantigene, nämlich Mimotope dieser Antigene.

Es handelt sich bei der als Antigen wirksamen Substanz um sogenannte Krebs- oder Tumorantigen-Mimotope mit der im Anspruch 1 definierten Sequenz. Dieses sind Substanzen natürlicher oder synthetischer Herkunft, die eine Immunantwort gegen Krebs bzw. Tumoren erzeugen. Es handelt sich bei den Krebs- oder Tumorantigen-Mimotopen um ein Peptid mit der Aminosäuresequenz Gln Met Trp Ala Pro Gln Trp Gly Pro Asp (Sequenznummer 1), das bei verschiedenen Krebsarten wie beispielsweise Brustkrebs auftritt, beispielsweise handelt es sich bei dem Mimotop um eines des HER2-neu Antigens.

Die Krebs- oder Tumorantigen-Mimotope können, müssen aber nicht, an einen Träger gekoppelt sein.

Bei einer gleichzeitigen Gabe von Arzneimitteln, die eine als Antigen wirksame Substanz gemäß Anspruch 1 und eine magensäurereduzierende Substanz umfassen, kann die als Antigen wirksame Substanz im Magen gleichzeitig oder mit zeitlicher Verzögerung im Vergleich zur magensäurereduzierenden Substanz abgegeben werden. Wichtig ist dabei nur, dass das Magenmilieu in der Weise eingestellt ist, dass die als Antigen wirksame Substanz ihre Wirkung bezüglich der Erzeugung einer Immunantwort entfalten kann. Pathophysiologisch oder iatrogen induziert können manche Individuen aus unterschiedlichen Gründen eine reduzierte Magensäure haben. Für die erfindungsgemäße Vakzinierung ist es jedoch notwendig, gezielt und kontrolliert den Säuregehalt zu beeinflussen. Insbesondere eignet sich die Einstellung des pH-Wertes des Magens auf einen pH-Wert im Bereich von 4 bis 7, bevorzugt von 4,3 bis 6,5, besonders bevorzugt von 4,8 bis 6. In diesem pH-Bereichen werden Pepsinogene nicht zur aktiven Protease konvertiert.

Das Mimotop bleibt daher unter hypoaziden Bedingungen konformationell intakt, wobei Konformationsepitope möglicherweise für die Induktion von IgE Antikörpern ausschlaggebend sind. Eine erhöhte Resorptionsrate des Mimotop durch die Magenwand konnte bisher noch nicht festgestellt werden, wird aber nicht ausgeschlossen.

Bevorzugt besteht die magensäurereduzierende Substanz aus einer die Magensäurebildung inhibierenden und/oder die Magensäure bindenden Substanz. Damit wird gewährleistet, dass die Art und Weise wie es zur Magensäurereduktion kommt auf unterschiedlichen Wirkmechanismen beruhen kann. Durch solche Mittel kann die Magensäurekonzentration auf die Bedürfnisse der erfindungsgemäßen Vakzinierung eingestellt werden.

Bevorzugt werden als magensäurereduzierende Mittel solche ausgewählt, die den Wirkstofffamilien der Antazida, H₂-Rezeptor-Antagonisten oder Protonenpumpeninhibitoren angehören. Durch das Verabreichen von Antazida, d.h. basischen Mitteln, wird vorhandene Magensäure reduziert. H₂-Rezeptor-Antagonisten inhibieren hingegen Histamin H₂-Rezeptoren kompetitiv und reversiv, wobei die Magensäurebildung vermindert wird. Protonenpumpeninhibitoren reduzieren ihrerseits die Magensäure durch direkte Beeinflussung der Säuresekretion.

Als Beispiele für Antazida seien folgende Wirkstoffe genannt: Natriumhydrogencarbonat, Calciumcarbonat, Magnesiumcarbonat, Magnesiumhydroxid und Magnesiumhydroxid-Gel, Magnesiumsilikat, Aluminiumphosphat, Aluminiumhydroxid und Aluminiumhydroxid-Gel, Hydrotalcite, Magaldrate, Dihydro-Aluminium-Natriumcarbonat, Magnesium-Aluminathydrat, Aminoessigsäure sowie Wismuth-Salze. Als protektiv über die Schleimhaut wirksame Substanzen seien Carbenoxolon und Sucralat (Aluminiumhydroxid und Saccharosesulfat) zu nennen. Als Beispiele für H₂-Rezeptorblocker seien exemplarisch genannt: Cimetidin, Ranitidin, Oxmetidin, Famotidin, Roxatidin und Nizatidin. Als Beispiele für Protonenpumpeninhibitoren seien genannt: Omezaprol, Lansoprazol, Pantoprazol und Rabeprazol. Da Anticholinergika u.a. eine Hemmung der Speichel- u. Magensaftabsonderung bewirken, können auch Vertreter dieser Klasse verwandt werden, so z.B. Pirenzepin.

Besonders bevorzugt werden solche magensäurereduzierenden Mittel aus den Mitteln Ranitidin-Hydrochlorid und Aluminium-Saccharose-Hydrogen-Sulfat ausgewählt. Beide Mittel sind für die erfindungsgemäßen Arzneimittel geeignet und im allgemeinen gut verträglich.

Als Antigen wirksame Substanzen werden vorzugsweise natürliche oder synthetische Antigen-Mimotope verwendet. Wie bereits erwähnt, werden Krebs- oder Tumorantigen-Mimotope bevorzugt. Besonders bevorzugt handelt es sich dabei um Peptide, die an einen Träger gekoppelt sein können. Die verwendeten natürlichen oder synthetischen Antigene bzw. Antigen-Mimotope oder Gemische davon bewirken nach der oralen Einnahme in Zusammenhang mit der magensäure-reduzierenden Substanz eine Immunantwort, die sich auf die Bildung von Immunglobulinen bezieht. Bevorzugt werden die Immunglobuline IgE und IgG1 gebildet.

In einer bevorzugten Ausführungsform wird das Antigen-Mimotop mit einem Träger konjugiert.

Als Träger eignen sich Moleküle, an die chemisch eines oder mehrere Antigen-Mimotope gebunden werden können.

Bevorzugt wird das Antigen-Mimotop mit einem immunogenen Träger konjugiert.

Solche Träger können Makromoleküle aller Art sein, die in der Lage sind immunstimmulierend zu wirken. Es ist jedoch von Bedeutung, dass ein gewählter Träger für Tiere und insbesondere für Menschen verträglich, d.h. nicht toxisch ist und keine Gefahren etwa eines Phagen oder Phagenpartikels bezüglich eventuell enthaltener Toxine oder der Möglichkeit der Infektion etwa von Darmbakterien in sich birgt, sowie nicht giftig ist und keine Serumkrankheiten oder Lebensmittelallergien auslöst. Die Konjugation mit einem Träger hat zur Folge, dass die Immunogenität der Vakzine erhöht wird. Als Beispiele von Trägern wären zu nennen Keyhole-Limpet-Hemocyanin (KLH), Tetanustoxoid (TT), Albumin-bindendes Protein (ABP) oder Rinderserumalbumin (BSA). Bevorzugt wird das Peptid oder dessen funktionelle Variante an Keyhole-Limpet-Hemocyanin (KLH) oder Tetanustoxoid (TT) konjugiert.

Bei einer weiteren bevorzugten Ausführungsform besteht der Träger aus einem Multimeren der Aminosäure Lysin. Dieses Polylysin ist bevorzugterweise als Dendrimer, d.h. als Polymer mit mehreren Verzweigungen aufgebaut, welches mehrere funktionelle Gruppen, insbesondere Aminogruppen, zur Verfügung stellt, so dass mehrere Peptide an den Träger gebunden werden können. Diese rein synthetische Variante wird als "multiple antigenic peptide" bezeichnet (MAP) und führt ebenfalls zur Erhöhung der Immunogenität ohne einen immunogenen Träger zu benutzen. Dies ist den Schriften von Tam JP, PNAS 1988; 85: 5409-13: "Synthetic peptide vaccine design: synthesis and properties of a highdensity multiple antigenic peptide" sowie von Olszewska W. et al. Virology 2000; 20: 98 - 105: "Protection against measles virus-induced encephalitis by anti-mimotope antibodies: the role of antibody affinity" nachzulesen.

Die Konjugation der Peptide an das Trägermaterial kann auf beliebige Weise erfolgen, beispielsweise auf gentechnologischem oder chemischem Weg, das heißt die Verknüpfung von Träger und einer funktionellen Gruppe am Peptid erfolgt durch eine chemische Reaktion. Bevorzugt findet sich die Verknüpfung an einem Ende des Peptides. Auf gentechnologischem Weg kann die Kopplung des Proteinträgermoleküls mit dem Peptid oder dessen Variante so hergestellt werden, dass eine für die Gesamtsequenz des Konjugates kodierende DNA- oder RNA-Sequenz in ein Expressionssystem eingebaut wird, von dem das Gesamtkonjugat dann exprimiert wird. Diese Form der Konjugation kann selbstverständlich nur für den Fall angewendet werden, dass auch das Gesamtkonjugat ein Proteinmolekül ist.

In einer weiteren bevorzugten Ausführungsform wird das Peptid über einen Linker mit einem Träger konjugiert. Dieser Linker dient zum einen als Abstandshalter zum Träger, aber auch zu einer verbesserten Kopplung an denselben. Vorzugsweise wird der Linker an die Peptidsequenz angefügt beziehungsweise mit ihr synthetisiert.

Als Linker eignet sich beispielsweise pentameres Glycin, das mit dem C-Terminus des Peptides verknüpft ist. Um die Kopplungsreaktion an ein Trägerprotein zu erleichtern, kann ein C-terminales Cystein eingeführt werden. Damit lässt sich das Peptid mit Linker über eine Disulfidbrücke an den Träger koppeln. Weiterhin eignet sich als Linker das Motiv GPGPG, welches ebenfalls durch einen Cysteinrest zum Motiv GPGPGC erweitert werden kann, um eine Disulfidbrückenbindung zum Trägerprotein zu erzielen.

Es ist weiterhin möglich, dass die Arzneimittel Tumor-Antigen-Mimotope als Monomere, Dimere, Trimere oder Oligomere enthalten, welche mit dem makromolekularen Träger konjugiert sind. Auch hier wird eine erhöhte Immunogenität erzeugt. Weiterhin ist bevorzugt, dass die monomeren, dimeren, trimeren oder oligomeren Tumor-Antigen-Mimotope einfach oder mehrfach an den makromolekularen Träger gebunden sind. Dadurch wird eine Steigerung der Immunogenität erzielt.

In Bezug auf die Einsetzbarkeit der als Antigen fungierenden Substanzen in den erfindungsgemäßen Arzneimitteln sind zahlreiche Möglichkeiten denkbar.

Die Arzneimittel besitzen die als Antigen wirksame Substanz gemäß Anspruch 1, die eine antitumorale Wirkung auslöst. Gerade im Bereich der Krebstherapie und Krebsvorsorge sind Vakzinierungen und insbesondere der Vorteil einer möglichen oralen Vakzinierung für die Patienten erwünscht.

Nachstehend sind Beispiele einer oralen Vakzinierung erläutert die zum Verständnis der Erfindung beitragen. Zur Erklärung zeigen:
- Figur 1:: Ein Balkendiagramm, mit dem IgE-Anstieg in verschiedenen Mausgruppen gegen als Antigen verwendeten Kaviar-Extrakt (Beispiel 1), gemessen im ELISA (Scala: OD-Werte), und
- Figur 2:: Ein Balkendiagramm, mit dem IgE-Anstieg in verschiedenen Mausgruppen gegen als Antigen verwendete Fischallergen Parvalbumin (Beispiel 2), gemessen im ELISA (Scala: OD-Werte).

### Beispiel 1:

Vier Gruppen (A bis D) zu je fünf Balb/c Mäusen wurden in folgender Weise behandelt:
- **Gruppe A:**: Orale Gabe von Zantac (ULSAL®, Wirkstoff: 20 microg Ranitidin pro Maus, einmalige Gabe pro Maus eine Stunde vor der Fütterung i.m. mit Antigen) und Antigen (2 mg Kaviar Protein Extrakt pro Maus),
- **Gruppe B:**: Orale Gabe einer Mischung von Ulcogant (Wirkstoff: 2 mg Sucralfat pro Maus)und Antigen (2 mg Kaviar Protein Extrakt pro Maus),
- **Gruppe C:**: Vergleichsbeispiel mit intraperitonealer Gabe von Kaviar Antigen (2 mg) und Aluminiumhydroxid als Adjuvans,
- **Gruppe D:**: Vergleichsbeispiel mit oraler Gabe des Antigens (2 mg).

Als Antigen wurde Kaviar-Extrakt verwendet. Als Referenz bezüglich des verwendeten Kaviar-Extraktes wird auf die Arbeit von Untersmayr E, Schöll I, Förster-Waldl E, Walter F, Riemer A, Boltz-Nitulescu G, Scheiner O, Jensen-Jarolim E. J Allergy and Immun. 2002; 109 (6): 1034-5 Bezug genommen. Das darin enthaltene Antigen weist ein Molekulargewicht von 118 kDa auf.

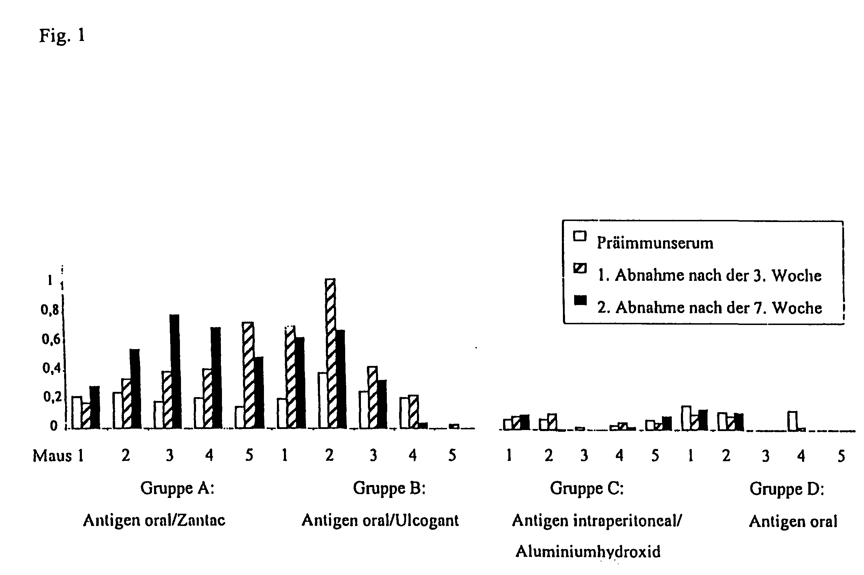

Die erhaltenen Resultate sind in Figur 1 wiedergegeben. Fig. 1 zeigt die IgE-Anstiege in den verschiedenen Mausgruppen gegen das Antigen, gemessen im ELISA (Skala: OD-Werte bei 405/450 nm). Den Gruppen A und B wurde erfindungsgemäß das Antigen in Verbindung mit einem magensäurereduzierenden Mittel oral verabreicht, während zum Vergleich Gruppe C nach einem Standardschema für IgE-Induktion immunisiert wurde und Gruppe D das Antigen ohne magensäurereduzierendes Mittel oral verabreicht wurde.

Die Befunde gemäß der vorliegenden Erfindung machen deutlich, dass eine Hypoazidität des Magens (durch Antazida-Behandlung) bei gleichzeitiger erstmaligen Einnahme eines Antigens zu einer IgE Induktion spezifisch gegen dieses neue Antigen führt.

Gängige Präparate, welche die Magensäurebildung inhibieren (Zantac® [Ranitidin-Hydrochlorid, GlaxoSmithKline]) oder welche die Magensäure binden und neutralisieren (Ulcogant® [Aluminium-Saccharose-Hydrogensulfat, Merck]), bewirken eine nachhaltige Induktion von IgE und auch IgG Antikörpern gegen ein gleichzeitig oder zeitlich verzögert gefüttertes neues Nahrungsmittel, in diesem Fall Kaviar-Extrakt, jedoch nicht gegen die tägliche Diät der Mäuse, gegen welche sie weiter tolerant blieben. Die erzielten IgE-Werte sind auch im Vergleich zu einer traditionellen Allergisierung auf intraperitonealem Weg mit Al(OH)₃ als Adjuvans hoch.

### Beispiel 2:

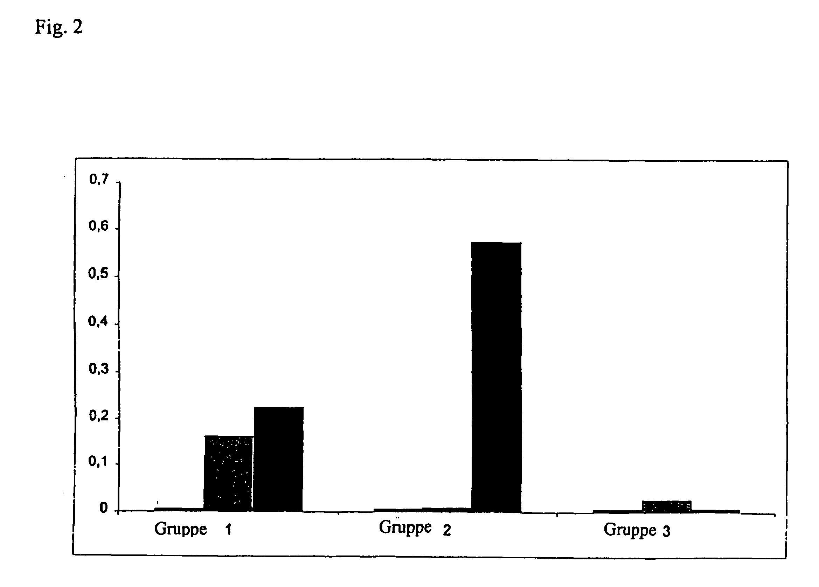

5-Wochen alte weibliche Balb/c Mäuse (n = 8 pro Gruppe) wurden durch intragastrale Fütterungen mit jeweils 250 microg/Maus rekombinanten Parvalbumin (Fischallergen, siehe Swoboda et al., J. of Immunology 2002; 168: 4576-84), behandelt. Gruppe 1 (siehe Fig. 2, x-Achse) wurde durch Zantac (Menge, Wirkstoff sh. Beispiel 1) i.m. eine Stunde vor der Fütterung vorbehandelt, Gruppe 2 mit Omeprazol i.m. (11,6 microg pro Maus, Losec, Fa. AstraZeneca) 2 Stunden vor der Fütterung, Gruppe drei erhielt nur Parvalbumin gefüttert ohne Vorbehandlung.

Die Tiere wurden am Tag 0 und 28 behandelt. Seren wurden am Tag 0 (jeweils erste Säulen), am Tag 28 (jeweils zweite Säule) und Tag 42 (jeweils dritte Säule) abgenommen und im ELISA getestet. Dazu wurden ELISA Platten mit recombinantem Parvalbumin beschichtet. Mausseren wurden 1:10 verdünnt und darauf über Nacht inkubiert. Nach Waschen wurde gebundenes Maus IgE mittels eines Peroxidase-markierten Rat anti-Maus IgE Antikörpers 1:1000 detektiert (PharMingen). Die Farbreaktion wurde durch Substratzugabe bewirkt und ist der Menge der spezifischen IgE Antikörper direkt proportional. Das Farbsignal wurde in einem ELISA Reader bei 405/450 nm gelesen (Fig. 2, y-Achse).

Fig. 2 zeigt die erhaltenen Resultate, wobei die im Diagramm dargestellten Werte die Mittelwerte der Mausgruppen (n = 8) sind. Es kann aus dem Beispiel des rekombinantes Parvalbumin gefolgert werden, dass Vorbehandlungen mit Antazida zu einer schnellen IgE Induktion gegen neue Nahrungs-Antigene führt.

Aufgrund der vermehrten Literaturberichte, welche auf eine anti-Tumorwirkung von IgE Antikörpern hinweisen (Reali, E.; Greiner, J.W.; Corti, A.; Gould, H.J., Bottazzoli, F.; Paganelli, G.; Schlom, J.; Siccardi, A.G.; Cancer Res., 2001; 61(14): 5517-22; Kershaw, M.H., Darcy, P.K.; Trapani, J.A.; MacGregor, D.; Smyth, m.J.; Oncol. Res., 1998, 10(3), 133-42; Neuchrist, C.; Kornfehl, J.; Grasl, M.; Lassmann, H.; Kraft, D.; Ehrenberger, K.; Scheiner, O.; Int. Archs. Allergy Immunol., 1994, 104: 97-100; Nagy, E.; Berczi, I.; Sehon, A.H.; Cancer Immunol. Immunother. 1991; 34(1): 63-9), kann aufgrund der Resultate der Beispiele 1 und 2 auch mit einem positiven Effekt einer Vakzinierung durch orale Applikation von Tumor-Antigen-Mimotop gemäß Anspruch 1 gerechnet werden.

## Patentansprüche

1. Arzneimittel, enthaltend eine als Tumorantigen wirksame Substanz und eine magensäurereduzierende Substanz als Kombinationspräparat zur gemeinsamen oder getrennten, gleichzeitigen oder zeitlich versetzten oralen Anwendung zur Vakzinierung gegen Krebs bzw. Tumoren, wobei das Tumorantigen als wirksame Substanz ein Antigen-Mimotop ist, das die Aminosäuresequenz Seq. 1: Gln Met Trp Ala Pro Gln Trp Gly Pro Asp hat.

2. Arzneimittel gemäß Anspruch 1, **dadurch gekennzeichnet, dass** das Antigen-Mimotope im Magen gleichzeitig oder mit zeitlicher Verzögerung im Vergleich zur magensäurereduzierenden Substanz freigesetzt wird.

3. Arzneimittel gemäß Anspruch 1 oder 2, **dadurch gekennzeichnet, dass** die magensäurereduzierende Substanz die Magensäurebildung inhibiert und/oder Magensäure bindet.

4. Arzneimittel gemäß Anspruch 3, **dadurch gekennzeichnet, dass** das magensäurereduzierende Mittel aus Mitteln der Wirkstofffamilien der Antazida, H₂-Rezeptor-Antagonisten und/oder Protonenpumpeninhibitoren ausgewählt werden.

5. Arzneimittel gemäß einem der vorstehenden Ansprüche, **dadurch gekennzeichnet, dass** der pH-Wert des Magens auf einen pH-Wert im Bereich von 4 bis 7 hinaufgesetzt wird.

6. Arzneimittel gemäß einem der vorstehenden Ansprüche, **dadurch gekennzeichnet, dass** die Antigen-Mimotope an einen Träger gekoppelt sind.

7. Arzneimittel gemäß einem der vorstehenden Ansprüche, **dadurch gekennzeichnet, dass** die Antigen-Mimotope als Monomere, Dimere, Trimere oder Oligomere mit dem Träger konjugiert sind.

8. Arzneimittel gemäß einem der vorstehenden Ansprüche, **dadurch gekennzeichnet, dass** die monomeren, dimeren, trimeren oder oligomeren Antigen-Mimotope einfach oder mehrfach an den Träger gebunden sind.

9. Arzneimittel nach einem der vorstehenden Ansprüche, **dadurch gekennzeichnet, dass** die Antigen-Mimotop eine antitumorale Wirkung auslösen.

## Claims

1. A drug containing a substance acting as a tumor antigen and a gastric acid reducing substance as a combination preparation for joint or separate, simultaneous or time shifted, oral application for vaccination against cancer or tumors, the tumor antigen being as the active substance an antigen mimotope having the amino acid sequence seq. 1: Gln Met Trp Ala Pro Gln Trp Gly Pro Asp.

2. The drug according to claim 1, **characterized in that** the antigen mimotope is released in the stomach simultaneously or with a time delay in comparison with the gastric acid reducing substance.

3. The drug according to claim 1 or 2, **characterized in that** the gastric acid reducing substance inhibits gastric acid formation and/or binds gastric acid.

4. The drug according to claim 3, **characterized in that** the gastric acid reducing agent is selected from agents of the families of active agents of antacids, H₂ receptor antagonists and/or proton pump inhibitors.

5. The drug according to any of the above claims, **characterized in that** the pH of the stomach is raised to a pH in the range of 4 to 7.

6. The drug according to any of the above claims, **characterized in that** the antigen mimotopes are coupled to a carrier.

7. The drug according to any of the above claims, **characterized in that** the antigen mimotopes are conjugated with the carrier as monomers, dimers, trimers or oligomers.

8. The drug according to any of the above claims, **characterized in that** the monomeric, dimeric, trimeric or oligomeric antigen mimotopes are bound to the carrier singly or multiply.

9. The drug according to any of the above claims, **characterized in that** the antigen mimotopes trigger an antitumoral effect.

## Revendications

1. Médicament contenant une substance agissant comme antigène tumoral en association avec une substance à activité d'antiacide gastrique, lesdites substances étant destinées, séparément ou ensemble, simultanément ou successivement, à une application orale pour effectuer une vaccination contre des cancers et/ou des tumeurs, l'antigène en tant que substance active étant un mimotope d'antigène avec la séquence d'acides aminés SEQ.IDNO :11: Gln-Met-Trp-Ala-Pro-Gln-Trp-Gly-Pro-Asp.

2. Médicament selon la revendication 1, **caractérisé en ce que** le mimotope d'antigène est libéré dans l'estomac simultanément avec la substance à activité d'antiacide gastrique ou bien consécutivement à cette dernière, au terme d'une période de retard.

3. Médicament selon la revendication 1 ou 2, **caractérisé en ce que** la substance à activité d'antiacide gastrique inhibe la formation d'acide gastrique et/ou neutralise l'acide gastrique.

4. Médicament selon la revendication 3, **caractérisé en ce que** la substance à activité d'antiacide gastrique est choisie dans les familles de principes actifs des antacides, des antagonistes du récepteur H₂ et/ou des inhibiteurs de pompe à protons.

5. Médicament selon l'une quelconque des revendications précédentes, **caractérisé en ce que** le pH de l'estomac est relevé de façon à se situer entre 4 et 7.

6. Médicament selon l'une quelconque des revendications précédentes, **caractérisé en ce que** les mimotopes d'antigène sont couplés à un support.

7. Médicament selon l'une quelconque des revendications précédentes, **caractérisé en ce que** les mimotopes d'antigène sont conjugués avec le support sous forme de monomères, de dimères, de trimères ou d'oligomères.

8. Médicament selon l'une quelconque des revendications précédentes, **caractérisé en ce que** les mimotopes d'antigène monomères, dimères, trimères ou oligomères sont liés une ou plusieurs fois au support.

9. Médicament selon l'une quelconque des revendications précédentes, **caractérisé en ce que** les mimotopes d'antigène déclenchent un effet antitumoral.
